# EUROPEAN PATENT SPECIFICATION

(11) **EP 1 690 878 B1**
(45) Date of publication and mention of the grant of the patent: **12.09.2007**
(21) Application number: 06250479.0
(22) Date of filing: 28.01.2006
(51) Int. Cl.: C08F 220/18

(54) **Thickener for aqueous systems**
Verdickungsmittel für wässrige Systeme
Epaississant pour systèmes aqueux

(30) Priority: 14.02.2005 US 652556 P
(43) Date of publication of application: 16.08.2006
(73) Proprietor: Rohm and Haas Company, Philadelphia, PA 19106-2399 (US)
(72) Inventor: Collin, Jennifer Reichl, Devon, Pennsylvania 19333 (US); Zeng, Fanwen, Belle Mead, NJ 08502 (US)
(74) Representative: Kent, Venetia Katherine

(56) References cited:
- EP-A- 0 839 843
- US-A- 5 631 317
- US-B1- 6 683 129

## Description

This invention relates to a polymeric thickener suitable for use in aqueous systems.

Rheology modifiers are used in aqueous cleaning products, including for example, shampoo, to increase viscosity at low shear rates while maintaining flow properties of the product at higher shear rates. In addition, rheology modifiers can provide effective, heat-age stable suspensions of particulate material or beads dispersed in the aqueous phase. A variety of copolymer thickeners made from vinyl monomers have been used for this purpose. For example, U.S. Pat. No. 6,635,702 discloses an acrylic emulsion polymer used as a thickener in aqueous systems. However, such thickeners often do not provide an optimum viscosity profile for a given application, or heat-age stable suspension of insoluble material.

The problem addressed by the present invention is the need for rheology-modifying polymers providing optimum viscosity profiles and/or good suspending properties.

### STATEMENT OF INVENTION

The present invention provides a polymer comprising: (a) from 25% to 45% carboxylic acid monomer residues; (b) from 50% to 65% C₂-C₄ alkyl (meth)acrylate residues; (c) from 2% to 20% of residues of at least one of: (i) an alkyl (meth)acrylate, (ii) a vinyl alkanoate, (iii) an N-vinyl alkylamide, and (iv) an N-alkyl (meth)acrylamide, wherein an alkyl group having from 6-18 carbon atoms is present; and (d) from 0.01% to 2% of residues of at least one crosslinker.

The present invention further provides an aqueous composition comprising from 0.1% to 8% of the aforementioned polymer.

### DETAILED DESCRIPTION

Percentages are weight percentages based on the entire composition, unless specified otherwise. As used herein the term "(meth)acrylic" refers to acrylic or methacrylic, and "(meth)acrylate" refers to acrylate or methacrylate, and the term "(meth)acrylamide" refers to acrylamide or methacrylamide. The term "acrylic polymers" refers to polymers of acrylic monomers, i.e., acrylic acid (AA), methacrylic acid (MAA) and their esters and amides, and copolymers comprising at least 50% of acrylic monomers. Esters of AA and MAA include, but are not limited to, methyl methacrylate (MMA), ethyl methacrylate (EMA), butyl methacrylate (BMA), hydroxyethyl methacrylate (HEMA), isobornyl methacrylate (IBOMA), methyl acrylate (MA), ethyl acrylate (EA), butyl acrylate (BA), ethylhexyl acrylate (EHA), and hydroxyethyl acrylate (HEA), as well as other alkyl esters of AA or MAA. Preferably, acrylic polymers have at least 75% of monomer residues derived from (meth)acrylic acid or (meth)acrylate or (meth)acrylamide monomers, more preferably at least 80%, and most preferably at least 88%.

For purposes of this invention, alkyl groups are straight or branched chain alkyl groups or aralkyl or alkyl carbocyclic groups, such as alkylphenyl groups. It is understood that the alkyl groups may be either of synthetic or of natural origin and, in the latter case particularly, may contain a range of chain lengths. For example, naturally sourced stearic acid, even of commercially pure quality may contain only about 90% of stearic chains, up to about 7% of palmitic chains and a proportion of other chains and lower quality products may contain substantially less stearic acid. It is intended herein that reference to the chain length of such groups is to the predominant chain length which is present as more than 50%, preferably in more than 75%, of the chains.

Preferably, the C₂-C₄ alkyl (meth)acrylate residues in the copolymer used in this invention are C₂-C₃ alkyl (meth)acrylate residues. Preferably, the C₂-C₄ alkyl (meth)acrylate residues are C₂-C₄ alkyl acrylate residues, more preferably C₂-C₃ alkyl acrylate residues and most preferably residues from EA. Preferably, the amount of C₂-C₄ alkyl (meth)acrylate residues is at least 52%, and most preferably at least 54%. Preferably, the amount of C₂-C₄ alkyl (meth)acrylate residues is no more than 62%, more preferably no more than 60% and most preferably no more than 58%.

A carboxylic acid monomer is any ethylenically unsaturated monomer having from 3-10 carbon atoms and containing at least one carboxylic acid group. Preferably, carboxylic acid monomers are selected from the group consisting of AA, MAA, itaconic acid, fumaric acid, crotonic acid and maleic acid; more preferably from AA and MAA, and most preferably MAA. Preferably, the amount of carboxylic acid monomer residues in the copolymer used in the present invention is at least 30%, more preferably at least 32% and most preferably at least 34%. Preferably, the amount of carboxylic acid monomer residues is no more than 40%, and most preferably no more than 38%.

The polymer of this invention comprises from 2% to 20% of residues of at least one of: (i) an alkyl (meth)acrylate, (ii) a vinyl alkanoate, (iii) an N-vinyl alkylamide, and (iv) an N-alkyl acrylamide; wherein an alkyl group having from 6-18 carbon atoms is present, i.e., the alkyl group of an alkyl (meth)acrylate, 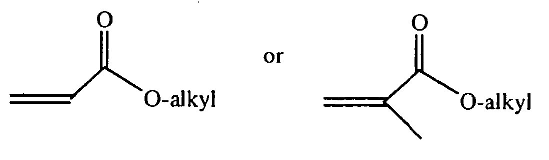 or the alkyl group of a vinyl alkanoate, 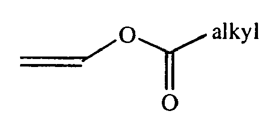 or the alkyl group of an N-vinyl alkylamide, 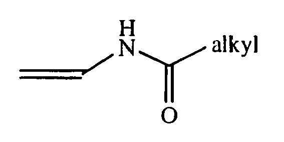 or the alkyl group of an N-alkyl (meth)acrylamide, 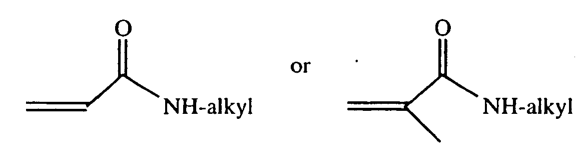 has from 6-18 carbon atoms. If more than one of (i), (ii), (iii) and (iv) are present, the total amount of such residues having a C₆-C₁₈ alkyl group is from 2% to 20%. Preferably, the polymer contains at least 3% of such residues, more preferably at least 5%, and most preferably at least 6%. Preferably, the polymer contains no more than 15% of such residues, and most preferably no more than 12%. Preferably, the polymer contains residues derived from an alkyl (meth)acrylate or a vinyl alkanoate. Preferably, the alkyl group has no more than 16 carbon atoms, more preferably no more than 12. Preferably, the alkyl groups are branched. Preferably, the alkyl (meth)acrylate is an alkyl acrylate.

In one preferred embodiment of the invention, the polymer contains residues of both an alkyl (meth)acrylate, in which the alkyl group has from 6-10 carbon atoms; and a vinyl alkanoate, in which the alkyl group has from 7-15 carbon atoms, more preferably 7-11 carbon atoms, and is branched. Preferably, both alkyl groups are branched. Preferably, the alkyl (meth)acrylate is an alkyl acrylate.

The aqueous compositions of the present invention contain from 0.1% to 8% of at least one polymer of this invention; i.e., the total amount of polymer(s) is in this range. Preferably, the amount of polymer in the aqueous composition is at least 0.5%, more preferably at least 0.75%, more preferably at least 1.0% and most preferably at least 1.2%. Preferably, the amount of copolymer in the aqueous composition is no more than 4%, more preferably no more than 3%, and most preferably no more than 2.5%. Preferably, the polymer is an acrylic polymer. The polymer, in aqueous dispersion or in the dry form, may be blended into an aqueous system to be thickened followed by a suitable addition of acidic or basic material if required.

Preferably, the pH of the aqueous composition is at least 3, more preferably at least 4. In one embodiment of the invention, the pH is at least 5, more preferably at least 5.5. Preferably, the pH is no more than 12, more preferably no more than 11, and most preferably no more than 8. In one embodiment of the invention, the pH is no more than 7. A variety of bases may be added to adjust the pH, e.g., a hydroxide base, such as sodium hydroxide, or an amine base, such as aminomethyl propanol. Preferably, a single pH adjustment is performed in formulating the aqueous composition, in contrast to the "back-titration" method described, e.g., in U.S. Pat. Nos. 4,529,773 and 6,635,702.

The aqueous compositions of the present invention preferably contain from 1% to 40% of at least one surfactant; i.e., the total amount of surfactant(s) is in this range. More preferably, the aqueous compositions of the present invention contain at least 5% of at least one surfactant, more preferably at least 10%, and most preferably at least 12%. Preferably, the aqueous composition contains no more than 25% of at least one surfactant, more preferably no more than 18%. Preferably, the aqueous composition has a pH from 3 to 12.

The surfactant(s) preferably is selected from the groups of anionic surfactants characterized by carboxylate, sulfonate, sulfate or phosphate solubilizing groups; nonionic surfactants characterized by amide or hydroxyl groups or ethylene oxide chains; and cationic, amphoteric or zwitterionic surfactants. Surfactants must be compatible with the thickening polymer and other ingredients of the aqueous system in the quantity required by the invention. Cationic surfactants characterized by amine or ammonium solubilizing groups, and/or amphoteric surfactants characterized by combinations of the anionic and cationic solubilizing groups may be selected. Preferred surfactants for use in the practice of the invention may be selected from the C₈ to C₁₈ fatty acids or their water soluble salts, water soluble sulfates or ether sulfates of C₈ to C₁₈ alcohols, sulfonated alkylaryl compounds such as, for example, dodecylbenzene sulfonate, alkylphenoxy polyethoxy ethanols, for example with C₇ to C₁₈ alkyl groups and 9 to 40 or more oxyethylene units, ethylene oxide derivatives of long chain carboxylic acids, for example of lauric, myristic, palmitic or oleic acids, ethylene oxide derivatives of long chain alcohols, for example of lauryl or cetyl alcohols, alkanolamides and polyglucosides, for example the alkyl polyglucosides, and surfactants derived from amino acids, e.g. glutamates. Suitable cationic surfactants may be, for example, lauryl pyridinium chloride, octylbenzyltrimethyl-ammonium chloride, dodecyl trimethylammonium chloride and ethylene oxide condensates of primary fatty acid amines. Suitable zwitterionic surfactants include, e.g., betaines.

The composition of the present invention optionally may include other ingredients, e.g., salts, co-rheology modifiers (e.g. Laponite^{™} clay, cellulosics, carrageenan, xanthan, PEG-150 distearate (Aculyn^{™} 60 rheology modifier), PEG-150 pentaerythrityl tetrastearate, other associative or non-associative acrylic rheology modifiers such as acrylates copolymer, derivatives of acrylates copolymer, Aculyn^{™} 33 rheology modifier, Aculyn^{™} 22 rheology modifier, Aculyn^{™} 28 rheology modifier, Acrylates/Beheneth-25 Methacrylate Copolymer, derivatives of Acrylates/Beheneth-25 Methacrylate Copolymer, and Aculyn^{™} 88 rheology modifier, Carbopol^{™} Aqua-SF1, Carbopol^{™} Aqua 30, and Carbopol^{™} Ultrez-21, other acrylic or urethane polymers like Aculyn^{™} 44 rheology modifier or Aculyn^{™} 46 rheology modifier, organic or inorganic particles (including, for example, abrasives, beads, mica, encapsulated oil beads), silicones, pearlizing agents, dispersed liquids, dispersants, soluble or dispersed biocides, vitamins, humectants, enzymes, bleach, emollient, fragrance, dyes, thioglycolic acid, UVA and UVB absorbers, infrared absorbers, etc. Insoluble materials which may be suspended in the aqueous composition include clay, beads, wax, gelatin and other particulate materials.

The polymer of the present invention is crosslinked, that is, at least one crosslinker, such as a monomer having two or more ethylenic unsaturated groups, is included with the copolymer components during polymerization. Preferred examples of such monomers include divinylbenzene, trimethylolpropane diallyl ether, tetraallyl pentaerythritol, triallyl pentaerythritol, diallyl pentaerythritol, diallyl phthalate, triallyl cyanurate, Bisphenol A diallyl ether, methylene bisacrylamide and allyl sucroses. In one embodiment, the crosslinker does not have ester or amide functionality. Divinylbenzene, trimethylolpropane diallyl ether (TMPDE) and tetraallyl pentaerythritol are especially preferred. The amount of crosslinker residue in the polymer is from 0.01% to 3%, based on the total weight of the monomers. Preferably, the amount of crosslinker residue in the polymer is no more than 1%, more preferably no more than 0.5%, and most preferably no more than 0.2%. Preferably the amount of crosslinker residue in the polymer is at least 0.05%, more preferably at least 0.1%. In one embodiment of the invention, the crosslinker is difunctional, e.g., divinylbenzene or TMPDE.

In one embodiment of the invention, the polymer is prepared in the presence of a chain transfer agent when a crosslinking agent is used. Examples of suitable chain transfer agents are carbon tetrachloride, bromoform, bromotrichloromethane, and compounds having a mercapto group, including 3-mercaptopropionic acid or long chain alkyl mercaptans and thioesters such as dodecyl-, octyl-, tetradecyl- or hexadecyl-mercaptans or butyl-, isooctyl- or dodecyl-thioglycolates. When used, the amount of chain transfer agent is typically from 0.01% to 5%, preferably from 0.1% to 1%, based on weight of the copolymer components. If the crosslinking agent is used in conjunction with a chain transfer agent, which are conflicting operations for polymerization purposes, not only is exceptional efficiency observed but also very high compatibility with hydrophilic surfactants, as manifested by increased product clarity.

The rheology modifier may be prepared by copolymerizing the monomers using known aqueous procedures at an acidic pH, inverse emulsification procedures at neutral pH, or precipitation or solution polymerization processes, and any other suitable additives known in the art, for example, a free-radical initiator such as peroxygen compounds or diazo compounds and, optionally, chain transfer agents. Suitable peroxygen compounds may be peroxides, hydroperoxides, persulfates or organic peroxides and a suitable quantity of initiator may be 0.01% to 3% by weight of the components of the copolymer. The copolymerization temperature may be about 25° C to 95° C, preferably 60° C to 92° C. The copolymer emulsion may be recovered by filtration and the copolymer may, if desired, be provided in dry form by spray drying or coagulation. U.S. Pat. Nos. 4,384,096, 4,663,385, 4,429,097 and 4,514,552 may be consulted for further general and specific details of suitable copolymerization and recovery techniques, and of suitable monomers and additives. The molecular weight of the primary polymer chain is typically in the range of about 100,000 to 1 million.

A particular aqueous composition in which the polymer of this invention is useful is a body wash. Typical components of a body wash, in addition to the polymer thickener and surfactant mentioned previously, include sufficient base or acid to attain a pH of 4-6.75, preferably 4.5-6.75, and more preferably 6.0-6.6; and optional ingredients, including silicones, pearlizing agents, vitamins, oils, fragrances, dyes, biocides, and insoluble beads made from a variety of materials, including polyolefins, e.g., polyethylene and polystyrene; gelatin; mica; encapsulated oil or vitamin beads; and Jojoba wax beads. Preferably, the amount of beads is from 0.1% to 2%, more preferably from 0.2% to 1%. Preferably, the average radius of the beads is from 0.1 mm to 10 mm. Typically, the surfactant used is a mixture of an anionic surfactant and an amphoteric surfactant, preferably from 8% to 16% of an anionic surfactant and from 1% to 5% of an amphoteric surfactant.

A second particular aqueous composition in which the polymer of this invention is useful is a shampoo. Typical components of a conditioning shampoo, in addition to the polymer thickener and surfactant mentioned previously, include sufficient base to attain a pH of 4-7, preferably 4.75-7.0. One particular embodiment of the invention is a conditioning shampoo containing a dispersed silicone, and optional ingredients, including pearlizing agents and zinc pyrithione or other anti-dandruff agents.

A third aqueous composition in which the polymer of this invention is useful is a hard surface cleaner. Typical components of a hard surface cleaner in addition to the polymer thickener and surfactant mentioned previously, include sufficient base to achieve a pH of 9-12, and optional ingredients including solvents, salts, fragrances, and dyes.

Other formulations in which the polymer is useful include hair gel (alcohol-containing and alcohol-free); hair styling cream, paste, or gum; shampoo, conditioner, 2 in 1 conditioning shampoo, body wash/shower gel, liquid soap, sunscreen lotions and sprays, tanning lotions, skin care lotions, skin care lotions containing vitamins, two-part hair dyes, permanent waving formulations, textile and hard surface cleaners, e.g., laundry detergent, liquid auto-dish detergent, manual dish detergent, spot-pretreaters, oven cleaners, and glass/window cleaners, and thickening all types of alcohol or water/alcohol formulations. The polymer may also be used as a polymeric emulsifier with or without co-emulsifiers or surfactants.

### EXAMPLES

### List of Polymers

| Entry | Polymer composition |
|---|---|
| A | 60EA/40MA·A//0.2DAP |
| B | 65EA/35MAA//0.2DAP |
| C | 59EA/5EHA/36MAA//0.2DAP |
| D | 59EA/5EHA/36MAA//0.116TMPDE |
| E | 54EA/36MAA/5EHA/5acrylamide//0.116TMPDE |
| F | 54EA/36MAA/5EHA/5styrene//0.116TMPDE |
| G | 55EA/5EHA/40MAA//0.2DAP |
| H | 50EA/10EHA/40MAA/0.2DAP |
| I | 45EA/15EHA/40MAA//0.2DAP |
| J | 55EA/5BA/40MAA//0.2DAP |
| | |
| K | 57EA/36MAA/7EHA//0.116TMPDE |
| L | 57EA/36MAA/7vinyl neodecanoate//0.116TMPDE |
| M | 54EA/36MAA/5EHA/5vinyl neodecanoate//0.116TMPDE |
| N | 56EA/36MAA/5EHA/3vinyl neodecanoate//0.116TMPDE |

| | |
|---|---|
| a. DAP is diallyl phthalate. b. TMPDE: trimethylolpropane diallyl ether | |

### Example 1. Preparation of Test Samples (Body Wash Formulation):

1. Weigh out sufficient polymer (A-N from table above) to have 2.0% polymer in final formulation. Predilute with deionized water.
2. Add sodium laureth sulfate (Steol^{™} CS-230, available from Stepan, Inc.) to have 12% active ingredient in final formulation.
3. Adjust pH of formulation to 6.2-6.4 with 10% NaOH solution.
4. Add cocamidopropyl betaine (Amphosol^{™} CA, available from Stepan, Inc.) to have 3% active ingredient in final formulation.
5. q.s. to total with deionized water. Allow sample to equilibrate for > 1h and adjust pH if necessary with NaOH.
6. Split sample into three portions. To one portion, add polyethylene beads (Performalene^{™} 2000 MiniPrills, available from New Phase Technologies) to 0.5% and mix to incorporate. To a second portion, add Jojoba wax beads (Jojoba Wax Prills 40/60, available from A&E Connock). The third portion will be used for rheology measurements.
7. Measure rheology of sample¹ at 25 °C and 40 °C. Monitor bead suspension at room temperature and 45 °C.
   ¹ Rheology analysis was conducted on a TA Instruments AR 2000 rheometer at 25 °C with a 60 mm 0.5° steel cone. A standard steady state flow from low to high shear stress method was used for analysis, with shear stress ramp from 0.002 Pa to 700 Pa. Viscosities (visc.) are reported in Pa·s at shear rates of 10⁻⁴ and 10 s⁻¹.

Results of clarity (clar.)(in NTU), viscosity and bead suspension testing are presented in the table below. A "Pass" result for bead suspension (bead sus.) indicates a stable suspension for more than 4 weeks at the test temperature. Polyethylene (PE) and Jojoba beads were used. Turbidity analysis was conducted using a HF Scientific Micro 100 Laboratory Turbidimeter, using specifications in EPA method 180.1 (Nephelometric Method). Heat age testing was performed by aging samples containing suspended beads in a FISHER ISOTEMP 300 Series Oven, Model 338F, with temperature controlled at 45 ± 2 °C. pH measurements were made with a METTLER TOLEDO SEVENEASY pH meter.

| poly. | clar. | visc., 25°C | | visc., 40°C | | bead sus., 25°C | | bead sus., 45°C | |
|---|---|---|---|---|---|---|---|---|---|
| | | 10⁻⁴ | 10 | 10⁻⁴ | 10 | Jojoba | PE | Jojoba | PE |
| A | 108 | 393 | 2 | 129 | 1 | Fail | Fail | Fail | Fail |
| B | 14 | 1134 | 2 | 503 | 1 | Pass | Pass | Fail | Fail |
| C | 52 | 1078 | 3 | 590 | 1 | Pass | Pass | Pass | Fail |
| D | 40 | 1133 | 4 | 874 | 1 | Pass | Pass | Pass | Pass |
| E | 38 | 1292 | 5 | 532 | 1 | Pass | Fail | Fail | Fail |
| F | 33 | 578 | 4 | 300 | 1 | Fail | Fail | Fail | Fail |
| G | 115 | 1269 | 4 | 612 | 1 | Pass | Pass | Pass | Fail |
| H | 50 | 991 | 6 | 782 | 1 | Pass | Pass | Pass | Fail |
| I | 70 | 1206 | 10 | 1272 | 2 | Pass | Pass | Pass | Fail |
| J | 116 | 1034 | 2 | 303 | 1 | Pass | Pass | Fail | Fail |
| | | | | | | | | | |
| K | 64 | 1912 | 4 | 1738 | 1 | Pass | Pass | Pass | Pass |
| L | 55 | 1824 | 6 | 1101 | 1 | Pass | Pass | Pass | Pass |
| M | 45 | 1643 | 5 | 1357 | 2 | Pass | Pass | Pass | Pass |
| N | 43 | 1577 | 6 | 1668 | 2 | Pass | Pass | Pass | Pass |

### Example 2. Pearlized 2-in-1 Conditioning Shampoo:

1. Weigh out sufficient polymer M to have 1.4% polymer in final formulation. Predilute with deionized water.
2. Add sodium laureth sulfate (Steol^{™} CS-230, available from Stepan, Inc.) to have 12% active ingredient in final formulation.
3. Adjust pH of formulation to 5.5-5.7 with 10% NaOH solution.
4. Add cocamidopropyl betaine (Amphosol^{™} CA, available from Stepan, Inc.) to have 3% active ingredient in final formulation.
5. Add a mixture of coco-glucoside, glycol distearate and glycerine (available as Euperlan^{™} PK-1200 from Cognis, Inc.) to have 2% active ingredient in final formulation.
6. Add a mixture of dimethicone, Laureth-23 and C12-15 Pareth-3 (Dow Corning 2-1491 Emulsion) to have 3% active ingredient in final formulation.
7. Add a 3:1 mixture of 5-chloro-2-methyl-4-isothiazolin-3-one and 2-methyl-4-isothiazolin-3-one (Kathon^{™} CG biocide, available from Rohm and Haas Company, Philadelphia, PA) to have 0.06% active ingredient in final formulation.
8. q.s. to total with deionized water. Allow sample to equilibrate for > 1h and adjust pH if necessary with NaOH.
9. Measure rheology of sample as described previously at 25 °C. Monitor sample for suspension/stabilization of silicone and pearlizing agent at room temperature and 45 °C.

The resulting pearlized viscous liquid was stable for at least two months at 25 °C and 45 °C. The apparent viscosity at a shear rate of 10 s⁻¹ was 3 Pa.s, and at 10⁻⁴ s⁻¹ was 800 Pa.s.

### Example 3. 2-in-1 Conditioning Shampoo:

1. Weigh out sufficient polymer M to have 1.4% polymer in final formulation. Predilute with deionized water.
2. Add ammonium lauryl sulfate (Stepanol^{™} AMV, available from Stepan, Inc.) to have 6.2% active ingredient in final formulation.
3. Add sodium laureth sulfate (Steol^{™} CS-230, available from Stepan, Inc.) to have 6.2% active ingredient in final formulation.
4. Adjust pH of formulation to 5.7-5.9 with 10% NaOH solution.
5. Add cocamidopropyl betaine (Amphosol^{™} CA, available from Stepan, Inc.) to have 3% active ingredient in final formulation.
6. Add a mixture of dimethicone, Laureth-23 and C12-15 Pareth-3 (Dow Corning 2-1491 Emulsion) to have 3% active ingredient in final formulation.
7. Add a 3:1 mixture of 5-chloro-2-methyl-4-isothiazolin-3-one and 2-methyl-4-isothiazolin-3-one (Kathon^{™} CG biocide, available from Rohm and Haas Company, Philadelphia, PA) to have 0.06% active ingredient in final formulation.
8. q.s. to total with deionized water. Allow sample to equilibrate for > 1h and adjust pH if necessary with NaOH.
9. Measure rheology of sample as described previously at 25 °C and 45 °C. Monitor sample for suspension/stabilization of silicone at room temperature and 45 °C.

The resulting opaque viscous liquid was stable for at least two months at 25 °C and 45 °C. The apparent viscosity at a shear rate of 10 s⁻¹ was 10 Pa.s, and at 10⁻⁴ s⁻¹ was 1450 Pa.s.

### Example 4. Low pH Body Wash:

1. Add sodium laureth sulfate (Steol^{™} CS-230, available from Stepan, Inc.) to have 12% active ingredient in final formulation to deionized water.
2. Adjust pH of mixture to 2.8 with 10% citric acid solution.
3. Weigh out sufficient polymer N to have 2.0% polymer in final formulation. Predilute with deionized water. Add to mixture.
4. Add cocamidopropyl betaine (Amphosol^{™} CA, available from Stepan, Inc.) to have 3% active ingredient in final formulation.
5. Adjust pH to 3.4 if necessary with 10% NaOH.
6. q.s. to total with deionized water. Allow sample to equilibrate for > 1h and adjust pH if necessary with NaOH.
   Split sample into three portions. To one portion, add polyethylene beads (Performalene^{™} 2000 MiniPrills, available from New Phase Technologies) to 0.5% and mix to incorporate. To a second portion, add Jojoba wax beads (Jojoba Wax Prills 40/60, available from A&E Connock). The third portion will be used for rheology measurements.
7. Measure rheology of sample as described previously at 25 °C and 40 °C and turbidity of the sample as described previously at 25 °C. Monitor sample for bead suspension at room temperature and 45 °C.

The apparent viscosity of the resulting mixture at a shear rate of 10 s⁻¹ was 70 Pa.s, and at 10⁻⁴ s⁻¹ was 5600 Pa.s. Turbidity of the sample was 59 ntu.

### Example 5. Hard Surface Cleaner:

1. Add C12-15 Pareth-7 (Neodol® 25-7, available from Shell Chemical) to have 2.5% active ingredient in the final formulation to deionized water.
2. Add sodium laureth sulfate (Steol® CS-230, available from Stepan, Inc.) to have 1% active ingredient in the final formulation.
3. Add potassium silicate to have 2.5% active ingredient in the final formulation.
4. Weigh out sufficient polymer N to have 2.0% polymer in final formulation. Predilute with deionized water and add to surfactant mixture.
5. Add PEG-400 to have 5% active ingredient in the final formulation.
6. Adjust pH of formulation to 10.9-11.1 with 10% NaOH solution.
7. q.s. to total with deionized water. Allow sample to equilibrate for > 1h.
8. Measure rheology of sample as described previously at 25 °C and 40 °C. The apparent viscosity at a shear rate of 10 s⁻¹ was 10, and at 10⁻⁴ s⁻¹ was 1300 Pa.s.

## Claims

1. A polymer comprising:
(a) from 25% to 45% carboxylic acid monomer residues;
(b) from 50% to 65% C₂-C₄ alkyl (meth)acrylate residues;
(c) from 2% to 20% of residues of at least one of: (i) an alkyl (meth)acrylate, (ii) a vinyl alkanoate, (iii) an N-vinyl alkylamide, and (iv) an N-alkyl (meth)acrylamide; wherein an alkyl group having from 6-18 carbon atoms is present; and
(d) from 0.01% to 2% of residues of at least one crosslinker.

2. The polymer of claim 1 comprising from 30% to 40% carboxylic acid monomer residues, wherein the carboxylic acid monomer residues are selected from acrylic acid and methacrylic acid.

3. The polymer of claim 2 comprising 52% to 62% C₂-C₄ alkyl acrylate residues.

4. The polymer of claim 3 comprising from 5% to 15% of residues of at least one of: (i) an alkyl acrylate, or (ii) a vinyl alkanoate, wherein an alkyl group having from 6-16 carbon atoms is present.

5. The polymer of claim 4 in which said at least one crosslinker is present at a level from 0.05% to 0.5%.

6. An aqueous composition comprising from 0.1% to 8% of the polymer of claim 5.

7. The aqueous composition of claim 6 having from 5% to 40% of at least one surfactant.

8. The aqueous composition of claim 7 having a pH from 3 to 12, and having from 5% to 25% of at least one surfactant.

9. The aqueous composition of claim 8, further comprising at least one of: (i) from 0.1% to 2% insoluble material; and (ii) 0.1 to 5% of silicone, pearlizing agent or dispersed liquids.

10. The aqueous composition of claim 9 having from 1% to 2.5% of said polymer.

## Patentansprüche

1. Polymer, umfassend:
(a) von 25% bis 45% Carbonsäuremonomerreste;
(b) von 50% bis 65% C₂-C₄ Alkyl(meth)acrylatreste;
(c) von 2% bis 20% an Resten von mindestens einem von: (i) einem Alkyl(meth)acrylat, (ii) einem Vinylalkanoat, (iii) einem N-Vinylalkylamid und (iv) einem N-Alkyl(meth)acrylamid, wobei eine Alkylgruppe mit von 6-18 Kohlenstoffatomen vorliegt; und
(d) von 0,01 % bis 2% an Resten von mindestens einem Vernetzungsmittel.

2. Polymer gemäß Anspruch 1, umfassend von 30% bis 40% Carbonsäuremonomerreste, wobei die Carbonsäuremonomerreste aus Acrylsäure und Methacrylsäure ausgewählt sind.

3. Polymer gemäß Anspruch 2, umfassend 52% bis 62% C₂-C₄ Alkylacrylatreste.

4. Polymer gemäß Anspruch 3, umfassend von 5% bis 15% an Resten von mindestens einem von (i) einem Alkylacrylat oder (ii) einem Vinylalkanoat, wobei ein Alkylrest mit von 6 bis 16 Kohlenstoffatomen vorliegt.

5. Polymer gemäß Anspruch 4, worin das mindestens eine Vernetzungsmittel in einem Anteil von 0,05% bis 0,5% vorliegt.

6. Wäßrige Zusammensetzung, umfassend von 0,1% bis 8% des Polymers gemäß Anspruch 5.

7. Wäßrige Zusammensetzung gemäß Anspruch 6, welche von 5% bis 40% von mindestens einem grenzflächenaktiven Mittel aufweist.

8. Wäßrige Zusammensetzung gemäß Anspruch 7, die einen pH von 3 bis 12 aufweist und von 5% bis 25% von mindestens einem grenzflächenaktiven Mittel aufweist.

9. Wäßrige Zusammensetzung gemäß Anspruch 8, weiter umfassend mindestens eines von: (i) von 0,1% bis 2% unlösliches Material und (ii) 0,1% bis 5% Silikon, Perlweißmittel oder dispergierten Flüssigkeiten.

10. Wäßrige Zusammensetzung gemäß Anspruch 9, welche von 1% bis 2,5% des Polymers aufweist.

## Revendications

1. Polymère comprenant :
(a) de 25 % à 45 % de résidus de monomère à acide carboxylique ;
(b) de 50 % à 65 % de résidus de (méth)acrylate d'alkyle en C₂-C₄ ;
(c) de 2 % à 20 % de résidus d'au moins un parmi : (i) un (méth)acrylate d'alkyle, (ii) un alcanoate de vinyle, (iii) un N-vinylalkylamide, et (iv) un N-alkyl(méth)acrylamide ; où un groupe alkyle ayant de 6 à 18 atomes de carbone est présent ; et
(d) de 0,01 % à 2 % de résidus d'au moins un réticulant.

2. Polymère selon la revendication 1, comprenant de 30 % à 40 % de résidus de monomère à acide carboxylique, où les résidus de monomère à acide carboxylique sont choisis parmi l'acide acrylique et l'acide méthacrylique.

3. Polymère selon la revendication 2, comprenant de 52 % à 62 % de résidus de (méth)acrylate d'alkyle en C₂-C₄.

4. Polymère selon la revendication 3, comprenant de 5 % à 15 % de résidus d'au moins un parmi : (i) un acrylate d'alkyle ou (ii) un alcanoate de vinyle, où un groupe alkyle ayant de 6 à 16 atomes de carbone est présent.

5. Polymère selon la revendication 4, dans lequel ledit au moins un réticulant est présent à un taux de 0,05 % à 0,5 %.

6. Composition aqueuse comprenant de 0,1 à 8 % de polymère selon la revendication 5.

7. Composition aqueuse selon la revendication 6, ayant de 5 % à 40 % d'au moins un tensioactif.

8. Composition aqueuse selon la revendication 7, ayant un pH de 3 à 12, et ayant de 5 % à 25 % d'au moins un tensioactif.

9. Composition aqueuse selon la revendication 8, comprenant en outre au moins un parmi : (i) de 0,1 % à 2 % de matière insoluble ; et (ii) de 0,1 % à 5 % de silicone, d'agent perlé ou de liquides dispersés.

10. Composition aqueuse selon la revendication 9, ayant de 1 % à 2,5 % dudit polymère.
